(19) Europäisches Patentamt
European Patent Office
Office européen des brevets

(11) Numéro de publication: **0 235 064 B1**

(12) # FASCICULE DE BREVET EUROPEEN

(45) Date de publication de fascicule du brevet: **17.06.92**

(51) Int. Cl.[5]: **A61K 7/42**, A61K 7/44, C07D 233/54, C07C 323/07, A61K 7/00, C07D 233/00

(21) Numéro de dépôt: **87440009.6**

(22) Date de dépôt: **13.02.87**

(54) **Composés aromatiques de structure amide dérivant d'acides amino-benzoiques, d'acides hydroxy-benzoiques, d'acides cinnamiques, d'acides urocaniques et de benzimidazoles, absorbant les UVB et/ou les UVA.**

(30) Priorité: **14.02.86 FR 8602125**

(43) Date de publication de la demande: **02.09.87 Bulletin 87/36**

(45) Mention de la délivrance du brevet: **17.06.92 Bulletin 92/25**

(84) Etats contractants désignés: **AT BE CH DE ES FR GB GR IT LI LU NL SE**

(56) Documents cités:
**DE-A- 1 443 923**
**DE-A- 2 932 923**
**DE-C- 957 162**
**FR-A- 1 184 710**
**FR-A- 2 204 628**
**FR-A- 2 579 461**
**FR-M- 2 470**

**CHEMICAL ABSTRACTS, vol. 101, no. 14, 1er octobre 1984, page 85, réf. no. 112584d Columbus, Ohio, US**

(73) Titulaire: **Robert, Dominique**
**248 avenue de la Vaugine**
**F-83300 Draguignan(FR)**

Titulaire: **Jung, Louis**
**205 route d'Oberhausbergen**
**F-67200 Strasbourg(FR)**

(72) Inventeur: **Robert, Dominique**
**248 avenue de la Vaugine**
**F-83300 Draguignan(FR)**
Inventeur: **Jung, Louis**
**205 route d'Oberhausbergen**
**F-67200 Strasbourg(FR)**

(74) Mandataire: **Nuss, Pierre**
**10, rue Jacques Kablé**
**F-67000 Strasbourg(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services

CHEMICAL ABSTRACTS, vol. 94, no. 19, 11.05.1981, page 692, réf. no. 157211h Columbus, Ohio, US; F. R. VAN HEERDEN et al.: "Isolation and synthesis of trans- and cis-(-)-clovamides and their deoxy analogs from the bark of Dalbergia melanoxylon"

CHEMICAL ABSTRACTS, vol. 73, no. 20, 16.11.1970, pages 340-341,réf. no. 109444f Columbus, Ohio, US; H. RIPPERGER et al.:"Putrescine amides from Kniphofia species"

CHEMICAL ABSTRACTS, vol 90, no. 10, 5 mars 1979, page 269, réf. no. 76372b Columbus, Ohio, US

CHEMICAL ABSTRACTS,vol. 105, no. 12, 22.09.1986, page 314, réf. no. 102334r Columbus, Ohio, US; M. F. SAETTONE et al.: "Substantivity of sunscreens: an apparaisal of some quaternary ammonium sunscreens"

CHEMICAL ABSTRACTS, vol. 85, no.7, 16.08.1976, page 486, réf. no.46109a Columbus, Ohio, US; H. L. HOLMES: "Preparation, properties, and reactions of some conjugated heteroenoid compounds and related compounds"

CHEMICAL ABSTRACTS, vol. 82, no. 18, 05.05.1975, page 287, réf. no. 116079c Columbus, Ohio, US

CHEMICAL ABSTRACTS, vol. 84, no. 22, 31.05.1976, page 346, réf. no. 155513c, Columbus, Ohio, US

J. MED. CHEM., vol. 18, no. 12, pages 1216-1223, Washington, D. C. US; Th. R. HERRIN et al.: "Antimalarials. Synthesis and antimalarial activity of 1-(4-methoxycinnamoye)-4-(5-phenyl-4-oxo--2-oxazolin-2-yl) piperazine and derivatives"

J. OF POLYMER SCIENCE: POL. CHEM. ED. vol. 21, 1983, pages 1111-1124 HIDETOMO ASHITAKA et al.: "Melt spinning of syndiotactic 1,2-polybutadiene for preparation of carbon fibers"

CHEMICAL ABSTRACTS, vol. 90, no. 7, 12.02.1979, page 641, réf. no. 55256b Columbus, Ohio, US; N. M. DIVANYAN et al.: "Synthesis and antiinflammatory activity of some salicylic acid amides-derivatives of alpha-amino acids"

J. CHEM. SOC., 1949, pages 1401-1406, chapitre 298 F. E. KING et al.: "Benziminazoles related to pteroic and pteroylglutamic acids"

CHEMICAL ABSTRACTS, vol. 73, no. 19, 9 novembre 1970, page 139, réf. no. 96092n, Columbus, Ohio, US; J.L. POUSSET et al.: "Isolation of urocanylglycine, a urinary derivative of histidine"

CHEMICAL ABSTRACTS, vol. 59, no. 7, 30 september 1963, colonnes 7316h-7317a

THE MERCK INDEX, An Encyclopedia of Chemical and Drugs, 9ième éd. 1976, page 58, Merck & Co. Inc. Rahway, N.J. US

JOURNAL OF PHARMACEUTICAL SCIENCES, Vol. 66, no. 6, juin 1977, pages 844-848, A. FUJII et al.: "Probiotics: Antistaphylococcal activity of 4-amino-cyclohexanecarboxylic acid, amino-benzoic acid, and their derivatives and structure-activity relation-ships"

CHEMICAL ABSTRACTS, vol. 82, no. 21, 26 mai 1975, page 630, réf. no. 140137t, Columbus, Ohio, US

## Description

La présente invention a pour objet la synthèse et/ou l'emploi de composés capables de s'opposer, sous forme de filtres solaires et éventuellement par induction de la mélanogénèse , aux effets nocifs du rayonnement solaire et aux réactions de photosensibilisation induites chez l'homme en particulier lors de l'emploi de certains médicaments ou de parfums ou de préparations cosmétiques.
Ce sont des composés aromatiques absorbant les UVB et/ou les UVA,substitués par une fonction amide ainsi que des composés dérivés obtenus par introduction d'une liaison éthylénique entre la structure aromatique et la fonction amide sans modification des caractéristiques spectrales.

Actuellement , une large gamme de préparations antisolaires a été développée pour la protection de la peau. Ces préparations contiennent à titre de produits actifs des composés capables d'absorber sélectivement les radiations nocives du soleil. Mais ces produits présentent l'inconvénient de se fixer difficilement à la surface de la peau et de s'éliminer au cours de leur utilisation ( par la sueur , au cours de bains de mer,...). De plus certains d'entre-eux passent dans la peau et dans le torrent circulatoire (D. CLAUS - Thèse de doctorat d'université de Strasbourg , mention Pharmacie- 1982);ils perdent ainsi leur activité et risquent d'être dangereux pour la santé de l'homme en ces d'utilisation prolongée . Les dérivés de l'acide para-méthoxycinnamique sont les filtres solaires les plus utilisés dans le monde. Pour retenir ce type de composés à la surface de la peau , une solution consiste à greffer sur leur structure chimique des groupements aminés isolés (Brevet français n° 81.08429 ), ou amino-acides ou peptidiques qui se fixent à la surface de la peau . Ainsi , récemment , deux séries de composés amino-acides de l'acide para-méthoxycinnamique et de l'acide trans-urocanique ont été synthétisées et leurs propriétés de filtres solaires revendiquées (Brevet français n° 84.10009 et Brevet français n° 85.04898).
Pour se protéger vis-à-vis des radiations solaires nocives, une autre solution consiste à stimuler le principal système protecteur naturel de la peau , la mélanogénèse. A l'heure actuelle aucun des produits utilisés comme filtres solaires ne revendique cette propriété.

On connaît du brevet français n° FR-1-A 184 710 des dérivés imidazoliques de formule générale :

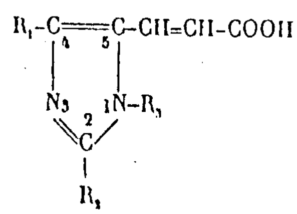

dans laquelle R1 représente un atome d'hydrogène ou un radical aliphatique, isocyclique ou hétérocyclique, R2 représente un atome d'hydrogène ou un groupe OH ou SH, R3 représente un radical aliphatique, isocyclique ou hétérocyclique, ainsi que leurs sels, esters, amides ou hydrazides. Cette formule ne mentionne, par conséquent, que la structure -COOH qui est acide au niveau de la peau.

On connaît également du document CHEMICAL ABSTRACTS, vol 84, n° 22, 31 mai 1976, page 346, réf. n° 155513c, Columbus, Ohio, US ; & JP-A-75 33 986 (AJINOMOTO CO. INC.) 2 avril 1975, un acide urocanique stearylamide de formule :

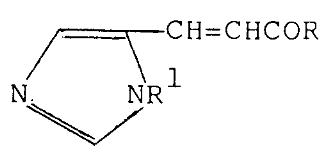

les dérivés $R = OR^2$ et $R = NR^3R^4$ étant utilisés notamment dans des compositions cosmétologiques, pharmaceutiques et alimentaires. Il s'agit, par conséquent, d'un acide urocanique et d'une stéarylamide, cette dernière n'étant ni un acide aminé, ni un peptide.

Les nouveaux composés, objets de l'invention , présentent les deux propriétés isolées ou conjointes nécessaires pour obtenir un maximum d'efficacité dans la protection solaire , c'est-à-dire une absorption prolongée des radiations ultra - violettes nocives par une meilleure adhérence cutanée du composé et l'induction de la mélanogénèse.
Les structures chimiques envisagées doivent pouvoir être solubilisées soit dans un solvant non aqueux, soit dans un solvant aqueux ; un caractère amphotère permettra une disposition à l'interface des globules d'une

émulsion.

Pour cela le même composé présentant l'une ou les deux propriétés photoprotectrices décrites précédemment existera soit sous la forme d'ester liposoluble ou présentera un groupement carboxylique libre , soit sous la forme d'ester à chaîne aminoalkyle ou à chaîne aminoaryle hydrosoluble, les chlorhydrates étant un exemple de sels d'amines hydrosolubles , soit sous la forme de sels hydrosolubles organiques ou minéraux de la fonction carboxylique libre. La fonction ester peut être replacée par une fonction amide.

Tous les composés , objets de l'invention , sont capables d'absorber les radiations ultra-violettes entre 360 nm et 260 nm avec un maximum qui peut se situer soit dans les UVA, soit dans les UVB, soit conjointement dans les UVA et UVB.

Les composés originaux décrits pour la première fois et dont l'invention est ici revendiquée sont des composés aromatiques absorbant les UVB et/ou les UVA substitués par une fonction amide , caractérisés en ce qu'ils répondent aux formules 1 à 3,

(1) 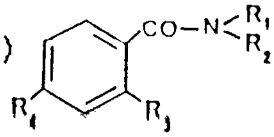

(2) 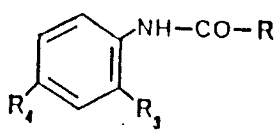

(3) 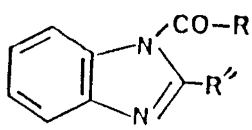

et en ce que , par l'introduction d'une liaison éthylènique entre la structure aromatique et la fonction amide sans modification des caractéristiques spectrales , on obtient les composés dérivés de formule 4 .

(4) 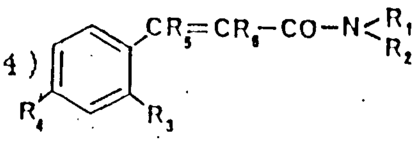

ces composés présentant au moins un chromophore couplé à une structure amide dont l'amine ou le groupement carbonyle est d'origine amino-acide ou peptidique, de manière à permettre une très bonne fixation au niveau de la peau, en particulier au niveau de l'épiderme, pouvant posséder des propriétés hydrosolubles et induisant la mélanogénèse,

R1 peut être l'hydrogène , un groupe alkyle , un groupe aryle.

R2 peut être un groupe:

de formule - CH - Y
| COOX

X et Y peuvent être l'hydrogène , un groupe alkyle, un groupe aryle , un groupe aminoalkyle ou aminoaryle

de formule - CH - $(CH_2)$ n - S - Z
| COOX

Z peut être l'hydrogène , un groupe alkyle, un groupe aryle , un groupe aminoalkyle ou aminoaryle.
X peut être l'hydrogène , un groupe alkyle , un groupe aryle , un groupe aminoalkyle ou aminoaryle.
n vaut 1 à 6, de préférence 1 ou 2.

de formule 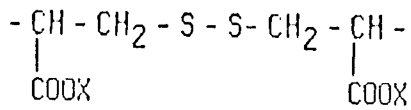

sur laquelle sont fixées deux molécules de chromophores filtrants présents dans les structures 1 à 4.
X peut être l'hydrogène, un groupe alkyle, un groupe aryle , un groupe aminoalkyle ou aminoaryle ou exister sous forme de sel minéral ou de sel organique. R1 peut aussi être égal à R2.

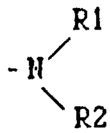

peut être l'acide urocanique ou un dérivé de l'acide urocanique de l'un des quatre types suivants:

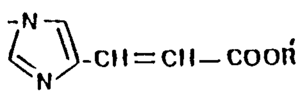

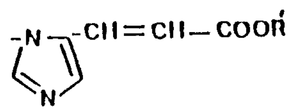

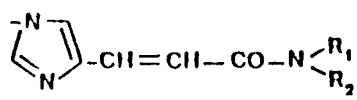

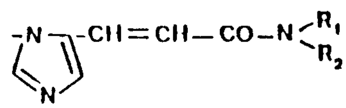

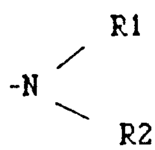

peut être une structure peptidique comprenant deux ou plusieurs acides aminés dont les fonctions acides ou amines terminales ou remifiées peuvent être libres ou engagées dans les groupements esters ou amides. R3 et R4 peuvent être un hydrogène , un groupe alcoxy de type CH3-O-, un groupe hydroxyle , un groupe acide , un groupe amine primaire , un groupe amide -NH-COR, un groupe ester de type -COOR'.
R3 et R4 peuvent être identiques ou différents.
R peut être:

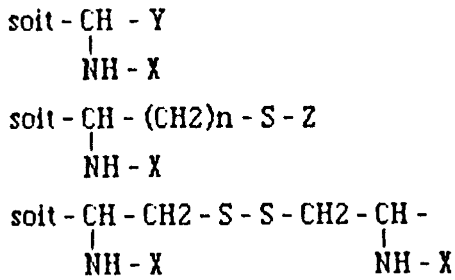

Z peut être l'hydrogène, un groupe alkyle, un groupe aryle. n vaut 1 à 6, de préférence 1 ou 2.

X et Y peuvent être l'hydrogène , un groupe alkyle linéaire ,ramifié , éventuellement hydroxylé ; un groupe aryle, un groupe aminoalkyle, un groupe aminoaryle.

R' peut être l'hydrogène , un groupe alkyle , un groupe aryle , un groupe aminoalkyle , un groupe aminoaryle , un sel organique ou minéral.

R" peut être un groupe alkyle , un groupe aryle , un groupe aminoalkyle , un groupe aminoaryle .

R5 et R6 peuvent être l'hydrogène , un groupe alkyle , un groupe aryle .

R5 et R6 peuvent être identiques ou différents .

Pour la structure 4 , sont à exclure les dérivés suivants :

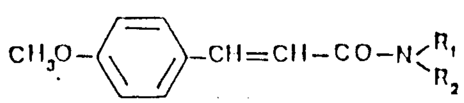

où $R_1$ et $R_2$ ont la même signification que pour les composés de formules 1 à 4 (Brevet français n° 85.04898)

Dans la présente description, le terme "alkyle" désigne des groupes hydrocarbonés aliphatiques contenant 1 à 12 atomes de carbone, à chaine droite ou ramifiée. On préfère les groupes alkyle inférieur, c'est-à-dire les groupes alkyles contenant 1 à 4 atomes de carbone. Le terme "alkyle" peut aussi désigner un groupe hydroxyalkyle.

Le terme "aryle" désigne les groupes aromatiques non hétérocycliques du type phényle, phénole, benzyle et les homologues supérieurs, substitués ou non ainsi que les groupes aromatiques hétérocycliques ayant 2 à 7 atomes de carbone dans le cycle aromatique, et 1 à 4 hétéroatomes pouvant être l'oxygène, l'azote, le soufre, du type furane, pyridine, oxazole.

Le terme "aminoalkyle" désigne des groupes hydrocarbonés aliphatiques contenant 1 à 12 atomes de carbone et 1 à 3 atomes d'azote , à chaine droite ou ramifiée. On préfère les groupements contenant 1 à 4 atomes de carbone et 1 atome d'azote.

Le terme "aminoaryle" désigne les substituants azotés de cycles aromatiques.

Le terme "sel minéral" désigne préférentiellement un sel soit de sodium , soit de potassium , soit de calcium.

Le terme de "sel organique" désigne préférentiellement un sel, obtenu par action d'une amine primaire ou secondaire ou tertiaire sur le groupement carboxylique ; on préfère soit un sel d'éthanolamine, soit un sel de pipéridine , soit un sel de pyrrolidine , soit un sel de pyridine ou leurs dérivés.

Parmi ces structures générales , sont revendiqués plus particulièrement les amides dus à la combinaison entre l'acide anthranilique ou l'acide para-aminobenzoïque ou l'acide salicyclique ou l'acide cyano-2 phényl-3 cinnamique et des acides aminés ou peptides non soufrés tels, par exemple , la tyrosine, l'histidine , l'acide glutamique , l'acide pyroglutamique ou des acides aminés ou peptides soufrés tels la méthionine, la cystéine, la S-méthylcystéine, la S-benzylcystéine, la cystine, le glutathion, le glutathion oxydé.

Parmi ces composés revendiqués se trouvent les composés répondant aux formules 1a, 1b , 1c et, 4a , qui sont dus à la combinaison entre l'acide anthranilique ou l'acide para-aminobenzoïque ou l'acide salicylique ou l'acide cyan-2 phényl-3 cinnamique ou l'acide para-méthoxycinnamique et une fonction amine d'une acide aminé ou d'un peptide.

(1a) 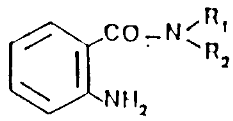

à l'exclusion du dérivé de l'ester méthylique de la glycine , R1 = H et R2 = - CH2 - COOCH3 (Späth, Kuffner, B.1934, 67, 1494) et à l'éxclusion du dérivé de la DL alanine,

R1 = H et R2 = 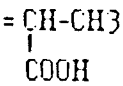

(Colles, Gibson, Soc. 1931 279, 282).

(1b) 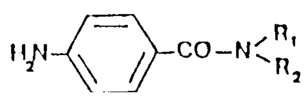

à l'exclusion des dérivés où

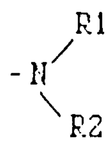

correspond à une structure - NH-CH2-COX' de glycine ou à une structure

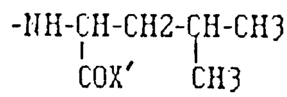

de leucine

X'correspondant à l'hydroxyle ou à une structure peptidique. (Landsteiner, Van der Scheer, J.exp.Med. 1939,69 ,705 et suivantes et idem 1934, 59, 769 et suivantes et idem 1932, 55, 781 et suivantes. Résumé Beilstein ed. Berlin, Amino-derivate der Monocarbonsaüren, E III 14, p. 1070 et 1071) et à l'exclusion du dérivé de la DL alanine, R1 = H et R2 = - CH(COOH)-CH3 et des dérivés où la DL alanine est remplacée soit par la DL valine, soit par la DL isoleucine, soit par la DL serine, soit par la DL méthionine, soit par l'acide aspartique. (Beilstein, ed.Berlin, Amino-dérivate der Monocarbonsaüren, EIII, 14 p.1071 à 1073.)

(1c) 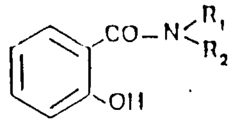

à l'exclusion des dérivés où

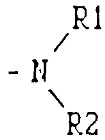

correspond à une structure - NH-CH2-COOX avec soit X = H (dérivé de la glycine), soit X = C2 H5 (dérivé de l'ester éthylique de la glycine) (Butler, Harington, Yuill, Biochem. J.1940, 34, 833- 840).
et à l'exclusion des dérivés où :

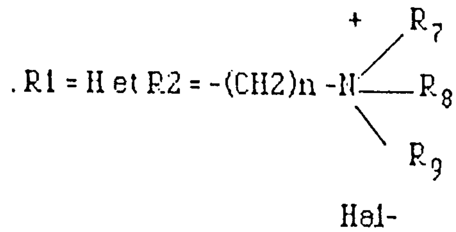

(Brevet U.S. n° =3,506,758)
.R1 = H et R2 = - $C_6H_5$ (Brevet U.S. n° = 2,874,090)
$Hal^-$ désigne un atome d'halogène, $Cl^-$ ,$Br^-$ .
R7 désigne un groupe alkyle ayant un à cinq atomes de carbone .
R8 désigne un groupe alkyle ayant un à cinq atomes de carbone .
R9 désigne un groupe alkyle ou un groupe benzylalkyle ayant trois à dix-huit atomes de carbone .
n est un entier de 2 à 12 .

(4a) 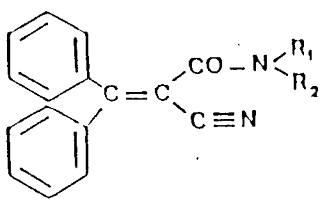

où R1 et R2 ont la même signification que dans les structures générales 1 à 4 .
Ces structures , qui correspondent donc à un chromophore couplé à une structure amide dont l'amine est d'origine amino-acide ou petidique , permettent une très bonne fixation au niveau de la peau , en particulier au niveau de l'epiderme .
C'est le cas notamment lorsque l'acide aminé ou le peptide greffé présente par ailleurs des groupements soufrés .
Sont revendiqués aussi plus particulièrement les produits répondent aux formules 2a, 2b et 3a caractérisés en ce qu'ils présentent un chromphore couplé à une structure amide dont le carbonyle est d'origine amino-acide ou peptidique . Ces structures permettent une très bonne fixation au niveau de la peau , en particulier au niveau de l'épiderme , par exemple avec l'acide pyroglutamique .
C'est aussi notamment le cas lorsque l'acide aminé ou le peptide greffé présente par ailleurs des groupements soufrés .

(2a) 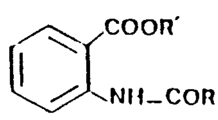

(2b) 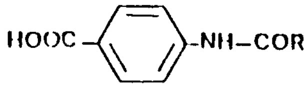

(3a) 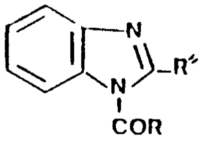

R, R' et R'' ont la même signification que dans les structures générales 1 à 4.
Sont revendiqués enfin plus particulièrement les produits répondant aux formules 1d et le caractérisés en ce qu'ils présentent une double structure amide de l'acide anthranilique ou de l'acide para-aminobenzoïque

(1d) 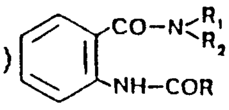

(1e) 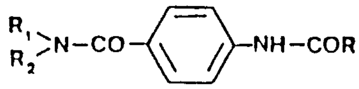

et les produits dus à la combinaison entre l'acide para-méthoxycinnamique et l'acide urocanique ou un de ses dérivés de l'une des deux formes suivantes :

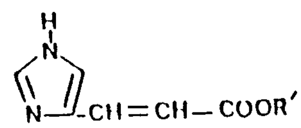

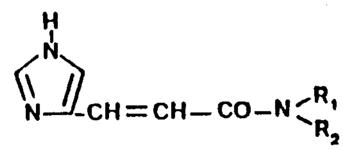

où R' et

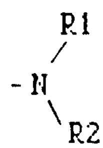

ont la même signification que dans la revendication 1 .

L'acide urocanique et ses dérivés peuvent se présenter sous forme trans ou forme cis . Ces derniers produits présentent deux groupements chromophores couplés qui peuvent posséder des propriétés hydrosolubles.

Toutes ces structures peuvent contenir des groupements aminoalkyles ou aminoaryles salifiés, par exemple sous forme de chlorhydrates, ou des sels organiques ou minéraux de la fonction carboxylique libre qui ajoutent une quatrième propriété d'hydrosolubilité , les autres composés étant plutôt liposolubles.

R1, R2, R et R' ont la même signification que dans les structures générales 1 à 4.

L'invention a aussi pour objet un procédé de préparation des dérivés tels que définis par les formules 1 à 4 ci-dessus.

Sous leur aspect général, les procédés de synthèse consistent à:

1) préparer le chlorure d'acide en faisant réagir, par exemple du chlorure de thionyle sur l'acide au sein d'un solvent organique.

2) faire réagir sur le chlorure d'acide précédent un composé à fonction amine primaire ou secondaire au sein d'un solvant organique en présence de triéthylamine.

3) salifier éventuellement par un agent alcalin minéral ou organique les composés présentant un groupement carboxylique libre.

4) salifier éventuellement par une base organique une fonction amine primaire ou secondaire ou tertiaire.

En particulier , il s'agira de faire réagir le chlorure d'acide anthranilique ou le chlorure d'acide para-aminobenzoïque ou le chlorure d'acide salicyclique ou le chlorure d'acide cyano-2 phényl-3 cinnamique sur une fonction amine d'un acide aminé ou d'un peptide soufrés ou non soufrés.

Pour certains composés, l'étape de formation du chlorure d'acide n'est pas nécessaire. La liaison peptidique peut être obtenue par combinaison entre la fonction carboxylique et la fonction amine en présence de dicyclohexylcarbodiimide ou d'un réactif similaire utilisé en synthèse peptidique.

Tous les composés de formules générales 1 à 4 sans exclusion ainsi que les composés dont la structure chimique et les propriétés de filtres solaires ont déjà été décrites et revendiquées dans les brevets français n° = 84.10009 et n° = 85.04898 sont capables d'induire la mélanogénèse d'une manière plus ou moins importante selon la structure de la molécule.

La propriété d'induction de la mélanogénèse est revendiquée pour tous les composés de formules 1 à 4

décrits.

Tous les composés de formules générales 1 à 4 sans exclusion sont aussi des filtres solaires caractérisés en ce qu'ils absorbent sélectivement les radiations U.V.B. et/ou U.V.A. et qu'ils se fixent au niveau des kératines pour les dérivés de la cystéine, de la cystine, de la méthionine et autres acides aminés et peptides soufrés . Ils peuvent induire la mélanogénèse et de ce fait posséder un triple rôle , à savoir filtrer sélectivement les radiations U.V.B. et/ou U.V.A., se fixer au niveau de l'épiderme de la peau et augmenter plus ou moins fortement la quantité de mélanines , substances à la base du principal système naturel de protection antisolaire . C'est le cas notamment des dérivés de l'acide trans-urocanique, de l'acide para-aminobenzoïque et de l'acide para-méthoxycinnamique où une forte induction de la mélanogénèse a pu être observée.

Les filtres qui comportent une structure amidique d'origine amino-acide ou peptidique présentent ainsi un caractère très substantif qui assure en plus du développement de la photoprotection naturelle , c'est-à-dire l'induction de la mélanogénèse , une excellente photoprotection artificielle pendant toute la durée d'application du topique. Les produits décrits sont particulièrement adaptés à un usage topique étant donné,comme mentionné plus haut , qu'ils ne dérivent quant à une partie de leur structure que de substances naturelles résidant normalement dans l'épiderme et qu'ils présentent de ce fait une tolérance et une inocuité parfaites.

Les acides aminés et peptides les plus utilisés sont :

- l'acide glutamique, l'acide pyroglutamique,
- des acides aminés aromatiques, en particulier la tyrosine, l'histidine,
- des acides aminés soufrés , en particulier la méthionine , la cystéine , la S-méthylcystéine , la S-benzylcystéine , la cystine, le glutathion, le glutathion oxydé,
- des acides aminés photoprotecteurs, en particulier l'acide urocanique. Ces exemples sont donnés à titre non limitatif.

Les composés, objets de l'invention, grâce à leurs propriétés, pourront donc être employés dans différents domaines.

En cosmétologie, les composés de formules 1 à 4 pourront être utilisés:

1°) en tant que filtres solaires pulsqu'ils présentent un bon spectre d'absorption ainsi qu'un caractére substantif marqué dû à leur structure qui leur permet d'assurer leurs fonctions pendant toute la durée de rétention au niveau de la peau.

2°) en tant qu'accélérateurs de bronzage par induction de la mélanogénèse. propriété que nous revendiquons pour tous les produits que nous décrivons ici, y compris l'acide urocanique. En fonction de la structure chimique, l'induction de la mélanogénèse est plus ou moins importante. Dans certains cas en particulier lors de la recherche du bronzage des inducteurs puissants seront préférés. Dans d'autres ces, en particulier lors d'applications localisées, des inducteurs moins puissants seront envisagés à plus forte concentration afin de faire prévaloir le pouvoir filtrant et la propriété de fixation aux kératines et aux tissus de la peau.

- Ces produits pourront également être utilisés dans l'industrie des parfums ou dans les préparations cosmétiques en particulier ceux contenant des parfums à base d'essence de bergamote. L'augmentation de la production de mélanines associée aux propriétés de filtre solaire s'opposent aux réactions de photosensibilisation dues à la synergie U.V.A. - bergeptène qui provoquent des photodermatoses de type dermite pigmentaire en breloque.

Deux types d'utilisations peuvent dans ce cas particulier être envisagés :

En cas de l'emploi du composé en tant que seul filtre solaire on choisira une concentration de l'ordre de 0,1 à 5% de la préparation afin d'éviter la formation de photodermatoses .

En cas de l'emploi du composé jouant conjointement le rôle de filtre solaire et d'inducteur de mélanogénèse, on choisira des concentrations de l'ordre de 1 à 10 %. La pigmentation locale observée est due à des mélanines dont la présence cutanée diminuera à l'arrêt de l'application.

Enfin le développement actuel des photodermatoses dues à des médicaments ou tout autre produit photosensibilisant conduit aussi à envisager l'utilisation par voie topique de ces produits dans l'industrie pharmaceutique. Une concentration plus élevée en mélanines au niveau de la peau et en présence d'une substance fixée aux kératines empêchant les radiations solaires d'interagir permettra avec sécurité l'emploi comme médicaments de certaines molécules responsables de photodermatoses. Parmi les médicaments induisant des réactions de photosensibilisation, on peut citer des photosensibilisants de contact tels que :

- des salicylanilides et leurs dérivés
- des sulfonamides et leurs dérivés
- des phénothiazines
- des teintures et colorants comme le rose Bengale
- le goudron de houille et certains de ses dérivés et différents produits comme la quinine , la trétinoine,

le péroxyde de benzoyle etc...

et des principes actifs administrés par voie générale à l'origine de photodermatoses tels :

- les psoralènes
- les tétracyclines
- les sulfonamides
- les sulfonylurées comme le tolbutamide
- les thiazides et sulfamides diurétiques
- l'acide nalidixique
- les antidépresseurs tricycliques comme l'amitriptyline et différents composés comme l'amiodarone, la griséofulvine, la quinidine, le méthotrexate, la dicarbazine, le fluorouracile et la vinblastine. Cette liste n'est pas limitative.

Afin de démontrer l'activité inductrice dans la synthèse des mélanines de différents produits, deux types d'essais ont été menés : d'une part un dosage global des mélanines extraites de cellules de mélanomes en culture, d'autre part un dosage des précurseurs des mélanines, notamment la dopa et la 5 S-cystéinyidopa.

La lignée cellulaire IGR 37 utilisée a été établie à l'institut Gustave Roussy à partir d'une tumeur métastasique humaine. Elle est cultivée en milieu essentiel minimum ( MEM ) additionné de 2 mM de glutamine, 9 µM de proline, 25 µM de vitamine C et 5 % de sérum de veau foetal. Le milieu est renouvelé 2 fois par semaine et les cultures sont repiquées en moyenne une fois par semaine dans des flacons de culture de 175 cm2 de surface. Les cultures sont maintenues à 37° C en atmosphère humide avec un taux de CO2 de 5 %.

Pour le dosage global des mélanines, nous avons opéré de la façon suivante : le premier jour , un million de cellules est introduit dans les flacons de culture contenant le millieu décrit précédemment. Le deuxième jour, le premier millieu est remplacé par du milieu contenant un des produits objets de l'invention ( milieu MEM+). Le quatrième jour, les cellules sont décollées avec une solution de trypsine et comptées avec un haemocytomètre. Les cellules sont ensuite centrifugées à 3000 tours/min pendant 10 minutes ; le surnageant est décanté et le culot cellulaire est stocké à - 20° C. Avant leur incorporation au milieu, les produits objets de l'invention sont dissous dans du DMSO. Les solutions sont filtrées stérilement sur des filtres de 0.22 µm de pore. Les concentrations efficaces retenues sont de l'ordre de 0,01 mM. Chaque solution de DMSO dans le milieu y est présente au taux maximum de 0,1 %.

Chaque expérience est réalisée sur un minimum de quatre flacons. Un minimum de quatre flacons témoins est cultivé dans les mêmes conditions en présence de 0,1 % de DMSO. Toutes les manipulations sont réalisées en absence d'irradiation ultra-violette ; les flacons et les solutions notamment sont protégés par du papier d'aluminium.

Le dosage est réalisé selon la méthode de WHITTAKER "changes in melanogenesis in cell culture" [Dev. Biol 8 , 99-127 (1963)]. Les culots cellulaires sont extraits trois fois avec de l'acide trichloracétique à 5 % à 4° C, deux fois avec un mélange éther-éthanol (1:3) à 4° C et une fois avec de l'éther absolu à température ambiante . Après séchage, le résidu est dissous dans de la soude 0,85 M et chauffé pendant 10 minutes à 100° C. Après refroidissement, la densité optique est mesurée à 400 nm avec un spectrophotomètre UV/visible . Le contenu en mélanines est exprimé en densité optique par cellule.

Résultats : l'adjonction des produits objets de l'invention dans le milieu de culture provoque une augmentation de la densité optique de l'ordre de 30 à 70 % par rapport aux flacons de cultures cellulaires non traités.

Pour la deuxième étude correspondant au dosage des précurseurs des mélanines, les cellules de mélanomes sont cultivées de la même façon que dans l'étude précédente Les culots cellulaires sont ensuite repris dans l'acide perchlorique 4N et centrifugés à 48000 g pendant 15 minutes à 4° C . Les surnageants sont récupérés directement dans les tubes à centrifuger contenant un mélange d'alumine pH 4 et d'éthylènediaminetétracétate de sodium . Chaque tube est ensuite ajusté à pH 8,6 (±0,01) avec de la soude 5N. Après 15 minutes d'agitation, les tubes sont centrifugés à 5600 t/min pendant 5 minutes à 4° C. Le surnageant est jeté et le mélange alumine-d'éthylènediaminetétracétate de sodium est lavé avec un tampon phosphate pH 8,6. L'opération est renouvelée deux fois. Aprés la deuxième centrifugation , le mélange alumine- éthylènediaminetétracétate de sodium est élué avec de l'acide perchlorique 0,5 N et agité pendant 15 minutes. Les tubes sont alors centrifugés à 4600 t/min pendant 5 minutes à 4° C. Le surnageant est prélevé et filtré . Le dosage est réalisé immédiatement ou bien le surnageant est congelé dans un tube à hémolyse pour un dosage ultérieur. Le dosage se fait par HPLC sur un appareil à détecteur UV/visible à 254 nm la colonne utilisée est de type ultraphère ODS de 3µm (4,6 mm x 7,5 cm). La phase mobile est constituée de :

- 95 % 3 mmoles d'acide orthophosphorique/1 d'eau
- 5% $CH_3OH$

Les précurseurs de mélanines dosés sont la dopa et la 5- S Cystéinyidopa (5-SCD). Des solutions témoins

de concentration 200 ng/ml sont réalisées avec de la dopa et de la 5 S-Cystéinyidopa
La dopa et la 5-SCD ont pour temps de rétention respectifs 2 minutes et 4 minutes 30. Résultats : Les cellules ayant été en contact avec les produits objets de l'invention présentent globalement une quantité en dopa et en 5-SCD supérieure d'environ 40 % dans les conditions expérimentales exposées.

Enfin chez l'homme, l'application d'une composition dermo-pharmaceutique contenant un de ces produits provoque une accélération du bronzage visible après six à huit jours par rapport aux zones non traitées.
Cette composition dermo-pharmaceutique combine le produit à un véhicule approprié, solution , émulsion , spray , est appliquée à la surface de la peau .
Deux exemples de préparation de nouveaux composés sont donnés ci-après à titre non limitatif.

EXEMPLE I

Synthèse de la N(amino-4 benzoyl)-L méthionine.

Dans un erlenmeyer, 1,35 g de chlorure d'acide para-aminobenzoïque sont dissous dans 20 ml de benzène en présence de 2,8 ml de triéthylamine. 1,49 de L-méthionine sont ajoutés progressivement et le mélange obtenu est filtré. Après évaporation à sec sous vide (15 mm Hg) du filtrat, le résidu est repris dans de l'acide chlohydrique N et extrait au chloroforme . La solution chloroformique est successivement lavée avec quelques ml de solution aqueuse de HKCO3 à 10 % et avec de l'eau. Elle est ensuite recueillie, séchée sur sulfate de magnésium et évaporée. Le produit obtenu est précipité dans du cyclohexane et filtré. Après recristallisation dans un mélange alcool 95° - eau (1 : 1) 1,95 g de cristaux blancs sont isolés .P.F:172 °C.

EXEMPLE II

Synthèse du N,N'-bis[[(méthoxy-4 cinnamoyl)-1 imidazolyl-4]-3 propène-2 oyl] L cystine dicarboxylate de méthyle.

*Première étape :*

Synthèse du N,N'-bis[(imidazolyl-4)-3 propène-2 oyl] L cystine dicarboxylate de méthyle.
Dans un erlenmeyer . 1.38 g d'acide (1H-imidazolyl-4)-3 propène-2 oïque. 2,06 g de dicyclohexylcarbodiimide (DCC) , 1,705 g de diester méthylique de L cystine et 10 ml de triéthylamine sont agités dans 50 ml de mélange acétonitrile-tétrahydrofurane 50/50 pendant trois heures . Le précipité de dicyclohexylurée obtenu est éliminé par filtration et le filtrat est évaporé à l'aide d'un évaporateur rotatif . Le résidu est repris dans de l'acide chlorhydrique N et extrait au chloroforme . La solution chloroformique est ensuite lavée avec quelques ml d'une solution aqueuse de HKCO3 à 10 % . Elle est ensuite recueillie, séchée sur sulfate de magnésium et évaporée. Le produit obtenu est précipité dans du cyclohexane et filtré.

*Deuxième étape :*

Dans un erlenmeyer, 1,016 g de l'amide obtenu lors de la première étape et 1 ml de triéthylamine sont mélangés dans 20ml de benzène anhydre . 784 mg de chlorure d'acide para-méthoxycinnamique sont additionnés . Après quelques minutes d'agitation , la solution est filtrée et évaporée à l'aide d'un évaporateur rotatif . Le résidu est repris dans de l'acide chlorhydrique N et extrait au chloroforme . La solution chloroformique est ensuite lavée avec quelques ml d'une solution aqueuse de HKCO3 à 10 % . Elle est ensuite recueillie, séchée sur sulfate de magnésium et évaporée. Le produit obtenu est précipité dans du cyclohexane et filtré. Après recristallisation dans un mélange éthanol 95°-eau (1:1), des cristaux de couleur jaune sont isolés .P.F:140°C.

**Revendications**

1. Composés aromatiques absorbant les UVB et/ou les UVA substitués par une fonction amide, caractérisés en ce qu'ils répondent aux formules 1 à 3 :

(1) 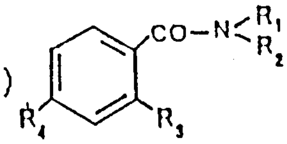

(2) 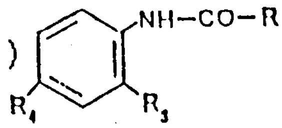

(3) 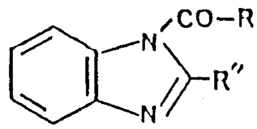

et en ce que, par introduction d'une liaison éthylènique entre la structure aromatique et la fonction amide sans modification des caractéristiques spectrales, on obtient les composés dérivés de formule 4

(4) 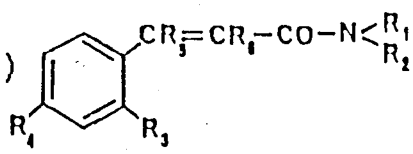

ces composés présentant au moins un chromophore couplé à une structure amide dont l'amine ou le groupement carbonyle est d'origine amino-acide ou peptidique, de manière à permettre une très bonne fixation au niveau de la peau, en particulier au niveau de l'épiderme, pouvant posséder des propriétés hydrosolubles et induisant la mélanogénèse,
R1 peut être l'hydrogène, un groupe alkyle, un groupe aryle,
R2 peut être un groupe :

de formule 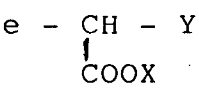

X et Y peuvent être l'hydrogène, un groupe alkyle, un groupe aryle, un groupe aminoalkyle ou aminoaryle

de formule 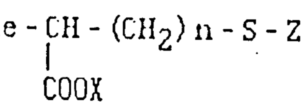

Z peut être l'hydrogène , un groupe alkyle , un groupe aryle , un groupe aminoalkyle ou aminoaryle
X peut être l'hydrogène, un groupe alkyle, un groupe aryle , un groupe aminoalkyle ou aminoaryle
n vaut 1 à 6 , de préférence 1 ou 2

de formule 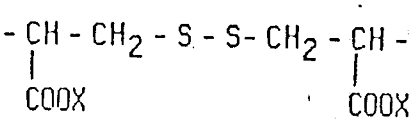

sur laquelle sont fixées deux molécules de chrompohores filtrants présents dans les structures 1 à 4
X peut être l'hydrogène, un groupe alkyle, un groupe aryle , un groupe aminoalkyle ou aminoaryle ou exister sous forme de sel minéral ou de sel organique

R1 peut aussi être égal à R2

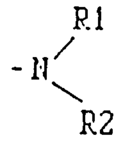

peut être l'acide urocanique ou un dérivé de l'acide urocanique de l'un des quatre types suivants :

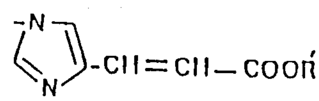

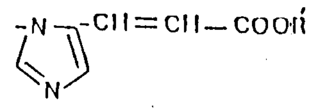

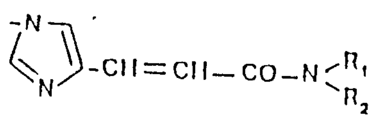

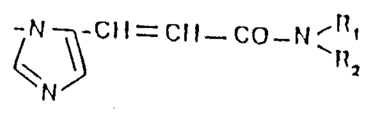

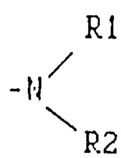

peut être une structure peptidique comprenant deux ou plusieurs acides aminés dont les fonctions acides ou amines terminales ou ramifiées peuvent être libres ou engagées dans les groupements esters ou amides R3 et R4 peuvent être un hydrogène , un groupe alcoxy de type CH3-O- , un groupe hydroxyle , un groupe acide , un groupe amine primaire , un groupe amide -NH-COR , un groupe ester de type -COOR'
R3 et R4 peuvent être identiques ou différents
R peut être :

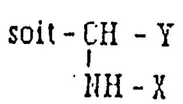

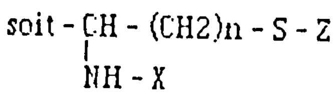

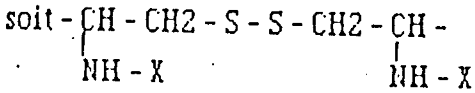

Z peut être l'hydrogène, un groupe alkyle, un groupe aryle
n vaut 1 à 6, de préférence 1 ou 2
X et Y peuvent être l'hydrogène, un groupe alkyle linéaire , ramifié , éventuellement hydroxylé ; un

groupe aryle, un groupe aminoalkyle, un groupe aminoaryle
R' peut être l'hydrogène , un groupe alkyle , un groupe aryle, un groupe aminoalkyle , un groupe aminoaryle , un sel organique ou minéral
R'' peut être un groupe alkyle, un groupe aryle, un groupe aminoalkyle, un groupe aminoaryle
R5 et R6 peuvent être l'hydrogène , un groupe alkyle , un groupe aryle
R5 et R6 peuvent être identiques ou différents
Pour la structure 4 , sont à exclure les dérivés suivants :

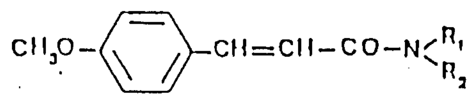

où R1 et R2 ont la même signification que pour les composés de formules 1 à 4.

2. Amides selon la revendication 1 dus à la combinaison entre l'acide anthranillique ou l'acide para-aminobenzoïque ou l'acide salicylique ou l'acide cyano-2 phényl-3 cinnamique et des acides aminés ou peptides non soufrés tels , par exemple , la tyrosine, l'histidine , l'acide glutamique , l'acide pyroglutamique ou des acides aminés ou peptides soufrés tels la méthionine , la cystéine , la S-méthylcystéine , la S-benzylcystéine , la cystine , le glutathion , le glutathion oxydé.

3. Amides selon l'une quelconque des revendications 1 à 2 , répondant aux formules 1a, 1b , 1c et 4a, dus à la combinaison entre l'acide anthranilique ou l'acide para-aminobenzoïque ou l'acide salicylique ou l'acide cyano-2 phényl-3 cinnamique ou l'acide para-méthoxycinnamique et une fonction amine d'un acide aminé ou d'un peptide

(1a) 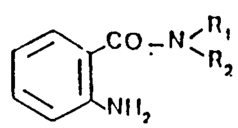

à l'exclusion du dérivé de l'ester méthylique de la glycine, R1 = H et R2 = - CH2 - COOCH3 et à l'exclusion du dérivé de la DL alanine,

R1 = H et R2 = 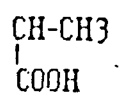

(1b) 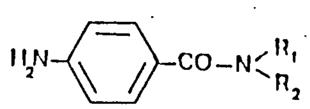

à l'exclusion des dérivés où

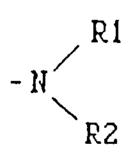

correspond à une structure - NH-CH2-COX' de glycine ou à une structure

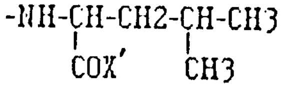

de leucine
X' correspondant à l'hydroxyle ou à une structure peptidique
et à l'exclusion du dérivé de la DL alanine, R1 = H et R2 = - CH(COOH)-CH3 et des dérivés où la DL alanine est remplacée soit par la DL valine, soit par la DL isoleucine, soit par la DL serine, soit par la DL méthionine, soit par l'acide aspartique

(1c) 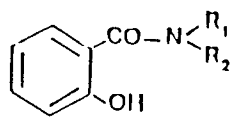

à l'exclusion des dérivés où

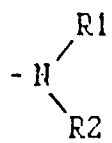

correspond à une structure - NH-CH2-COOX avec soit X = H (dérivé de la glycine), soit X = C2 H5 (dérivé de l'ester éthylique de la glycine)
et à l'exclusion des dérivés où:

.R1 = H et R2 = 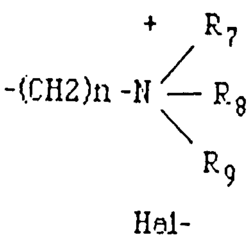

.R1 = H et R2 = - $C_6$ $H_5$
$Hal^-$ désigne un atome d'halogène , $Cl^-$ , $Br^-$
R7 désigne un groupe alkyle ayant un à cinq atomes de carbone
R8 désigne un groupe alkyle ayant un à cinq atomes de carbone
R9 désigne un groupe alkyle ou un groupe benzylalkyle ayant trois à dix-huit atomes de carbone
n est un entier de 2 à 12

(4a) 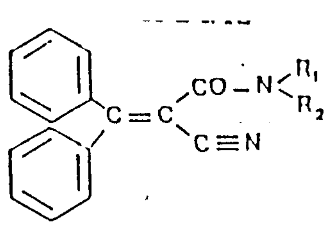

où R1 et R2 ont la même signification que dans la revendication 1 .

**4.** Produits selon l'une quelconque des revendications 1 à 3 présentant un chromophore couplé à une structure amide dont l'amine est d'origine amino-acide ou peptidique présentant par ailleurs des groupements soufrés, ces structures permettant une très bonne fixation au niveau de la peau, en

particulier au niveau de l'épiderme.

5. Produits selon l'une quelconque des revendications 1 à 2 caractérisés en ce qu'ils répondent aux formules 2a , 2b , 3a et en ce qu'ils présentent un chromophore couplé à une structure amide dont le carbonyle est d'origine amino-acide ou peptidique , ces structures permettant une très bonne fixation au niveau de la peau, en particulier au niveau de l'épiderme , par exemple avec l'acide pyroglutamique

(2a) 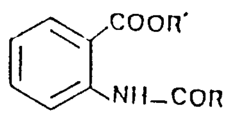

(2b) 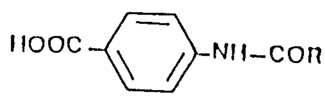

(3a) 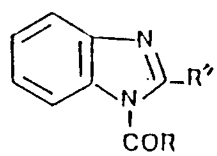

où R , R' et R'' ont la même signification que dans la revendication 1 .

6. Produits selon l'une quelconque des revendications 1 à 2 et 5 présentant un chromophore couplé à une structure amide dont le carbonyle est d'origine amino-acide ou peptidique présentant par ailleurs des groupements soufrés , ces structures permettant une très bonne fixation au niveau de la peau en particulier au niveau de l'épiderme .

7. Produits selon l'une quelconque des revendications 1 à 2 caractérisés en ce qu'ils répondent aux formules 1d et 1e et présentent une double structure amide de l'acide anthranilique ou de l'acide para-aminobenzoïque à l'origine d'une très bonne fixation au niveau de la peau, en particulier au niveau de l'épiderme

(1d) 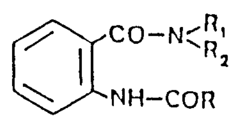

(1e) 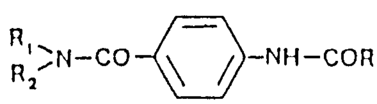

où R1, R2, R ont la même signification que dans la revendication 1 .

8. Amides selon la revendication 1 dus à la combinaison entre l'acide para-méthoxycinnamique et l'acide urocanique ou un de ses dérivés de l'une des deux formes suivantes :

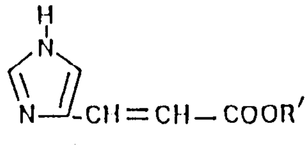

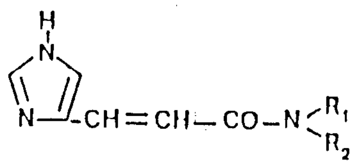

où R' et

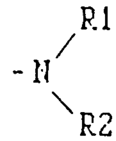

ont la même signification que dans la revendication 1 .

**9.** Produits selon la revendication 8 présentant deux groupements chromophores couplés et possédant des propriétés hydrosolubles .

**10.** Structures selon l'une quelconque des revendications 1 à 9 contenant des groupements aminoalkyles ou aminoaryles salifiés, par exemple sous forme de chlorhydrates, ou des sels organiques ou minéraux de la fonction carboxylique libre pour ajouter une quatrième propriété d'hydroxolubilité , les autres composés étant plutôt liposolubles.

**11.** Procédé pour l'obtention des dérivés selon l'une quelconque des revendications 1 à 4caractérisé en ce qu'il consiste à faire réagir le chlorure d'acide anthranilique ou le chlorure d'acide para-aminobenzoïque ou le chlorure d'acide salicylique ou le chlorure d'acide cyano-2 phényl-3 cinnamique sur une fonction amine d'un acide aminé ou d'un peptide soufrés ou non soufrés.

**12.** Procédé pour l'obtention des dérivés selon l'une quelconque des revendications 1 à 10, caractérisé en ce qu'il consiste à obtenir directement la liaison amidique par combinaison entre la fonction carboxylique et la fonction amine en présence de dicyclohexylcarbodiimide ou d'un réactif similaire utilisé en synthèse peptidique.

**13.** Utilisation des composés selon l'une quelconque des revendications 1 à 10 comme filtre solaire absorbant sélectivement les radiations U.V.B. et/ou U.V.A., se fixant au niveau des kératines pour les dérivés de la cystéine, de la cystine, de la méthionine et autres acides aminés et peptides soufrés et pouvant induire la mélanogénèse et de ce fait posséder un triple rôle, à savoir filtrer sélectivement les radiations U.V.B. et/ou U.V.A., se fixer au niveau de l'épiderme de la peau et augmenter plus ou moins fortement la quantité de mélanines, substances à la base du principal système naturel de protection antisolaire.

**14.** Utilisation des composés selon l'une quelconque des revendications 1 à 10 comme accélérateurs de bronzage.

**15.** Utilisation des composés selon l'une quelconque des revendications 1 à 10, à titre d'ingrédient actif comme compositions dermo-pharmaceutiques ou cosmétologiques en combinaison avec un véhicule approprié, solution, émulsion, spray par exemple, le tout étant à appliquer à la surface de la peau de l'homme.

**16.** Utilisation des composés selon l'une quelconque des revendications 1 à 10 en tant que photoprotecteurs pour l'industrie des parfums et des cosmétiques ou pour l'industrie pharmaceutique pour les médicaments à l'origine de photodermatoses, notamment :

- les topiques médicamenteux contenant des psoralènes ou leurs dérivés, des salicylanilides ou leurs dérivés, des sulfonamides ou leurs dérivés, des phénothiazines, des teintures ou colorants comme le rose Bengale, du goudron de houille ou certains de ses dérivés et enfin différents

produits comme la quinine, la trétinoïne, le péroxyde de benzoyle...

- les médicaments administrés par voie générale à l'origine de photodermatoses tels les psoralènes, les tétracyclines, les sulfonamides, les sulfonylurées comme le tolbutamide, les thiazides et sulfamides diurétiques, l'acide nalidixique, les antidépresseurs tricycliques comme l'amitriptyline et différents composés comme l'amiodarone, la griséofulvine, la quinidine, le méthotrexate, la dicarbazine, le 5 fluorouracile et la vinblastine.

## Claims

**1.** Aromatic compounds absorbing the UVB and/or the UVA substituted by an amide function, characterised in that they correspond to formulae 1 to 3:

(1) 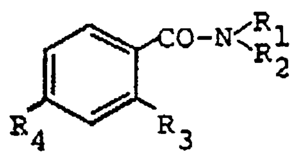

(2) 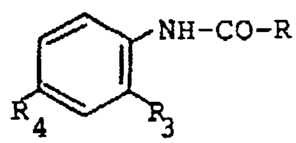

(3) 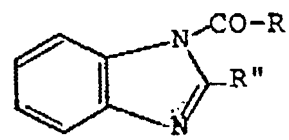

and in that, by introduction of an ethylene bond between the aromatic structure and the amide function without modification of the spectral characteristics, derived compounds corresponding to formula 4 are obtained

(4) 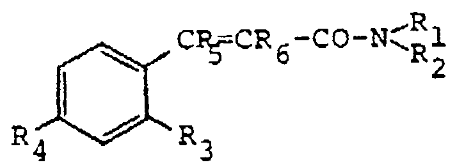

these compounds having at least one chromophore coupled to an amide structure of which the amine or the carbonyl group is Of amino-acid or peptide origin so as to allow very good fixing in the region of the skin, in particular in the region of the epidermis, and being able to have water-soluble properties and inducing melanogeneois,

R1 may be hydrogen, an alkyl group, an aryl group,

R2 may be a group of formula

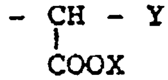

X and Y may be hydrogen, an alkyl group, an aryl group, an aminoalkyl or aminoaryl group

of formula 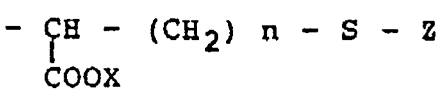

Z may be hydrogen, an alkyl group, an aryl group, an aminoalkyl or aminoaryl group,
X may be hydrogen, an alkyl group, an aryl group, an aminoalkyl or amino-aryl group
n is equal to 1 to 6, preferably 1 or 2,

of formula 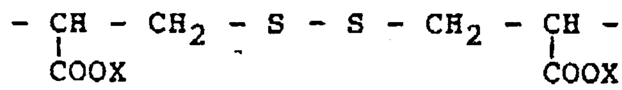

on which there are fixed two molecules of filtering chromophores present in structures 1 to 4
X may be hydrogen, an alkyl group, an aryl group, an aminoalkyl or aminoaryl group or may exist in the form of a mineral salt or organic salt
R1 may also be identical to R2

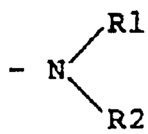

may be urocanic acid or a derivative of urocanic acid of one of the following four types:

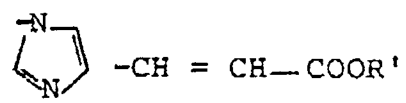

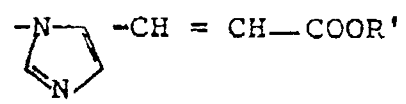

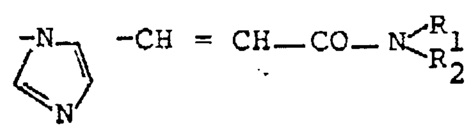

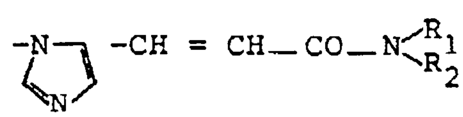

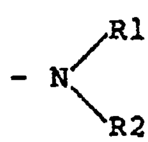

may be a peptide structure comprising two or more amino-acids of which the terminal or branched acid or amine functions may be free or bound in ester or amide groups
R3 and R4 may be a hydrogen, a CH3-0- type alkoxy group, a hydroxyl group, an acid group, a primary amine group, a -NH-COR amide group, a -COOR'-type ester group
R3 and R4 may be the same or different
R may be:

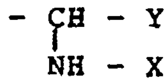

or 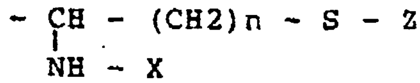

or 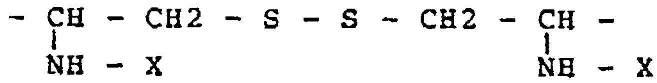

Z may be hydrogen, an alkyl group, an aryl group
n is equal to 1 to 6, preferably 1 or 2
X and Y may be hydrogen, a linear, branched, and optionally hydroxylated alkyl group, an aryl group, an aminoalkyl group, an aminoaryl group.
R' may be hydrogen, an alkyl group, an aryl group, an aminoalkyl group, an aminoaryl group, an organic or mineral salt.
R" may be an alkyl group, an aryl group, an aminoalkyl group, an aminoaryl group
R5 and R6 may be hydrogen, an alkyl group, an aryl group
R5 and R6 may be the same or different
The following derivatives are to be excluded for structure 4

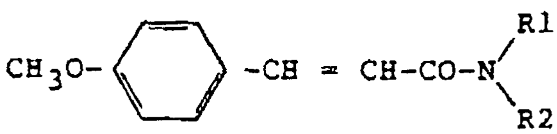

wherein R1 and R2 have the same meaning as for the compounds corresponding to formulae 1 to 4.

2. Amides according to claim 1, formed by the combination between anthranilic acid or para-aminobenzoic acid or salicylic acid or 3-phenyl-2-cyano cinnamic acid and non-sulphur-containingamino-acids or peptides such as, for example, tyrosine, histidine, glutamic acid, pyroglutamic acid or sulphur-containing amino-acids or peptides such as methionine, cysteine, S-methylcysteine, S-benzylcysteine, cystine, glutathione, oxydised glutathione.

3. Amides according to any one of claims 1 to 2, corresponding to formulae 1a, 1b, 1c and 4a, formed by the combination between anthranilic acid or para-aminobenzoic acid or salicylic acid or 3-phenyl-2-cyano cinnamic acid or paramethoxycinnamic acid and an amine function of an amino-acid or a peptide

(1a) 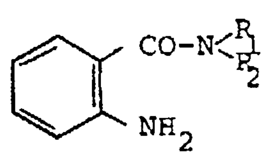

to the exclusion of the methyl ester derivative of glycine R1 = H and R2 = - CH2 - COOCH3 and to the exclusion of the DL alanine derivative ,

R1 = H and R2 = 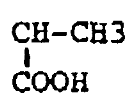

(1b) 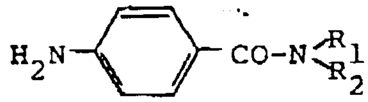

to the exclusion of the derivatives where

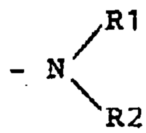

corresponds to a -NH-CH2-COX' structure of glycine or a

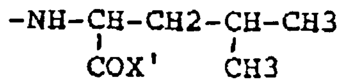

structure of leucine
X' corresponding to hydroxyl or to a peptide structure and to the exclusion of the DL alanine derivative, R1 = H and R2 = -CH(COOH)-CH3 and derivatives where the DL alanine is replaced by DL valine, or by DL isoleucine or by DL serine, or by DL methionine, or by aspartic acid

(1c) 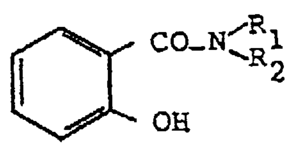

to the exclusion of the derivatives where

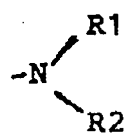

corresponds to a -NH-CH2-COOX structure wherein either X = H (glycine derivative) or X = C2H5 (the ethylester derivative of glycine)
and to the exclusion of the derivatives where:

R1 = H and R2 = -(CH2)n - 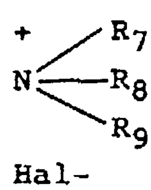

R1 = H and R2 = $-C_6H_5$
$Hal^-$ represents a halogen atom, $Cl^-$, $Br^-$
R7 represents an alkyl group having one to five carbon atoms
R8 represents an alkyl group having one to five carbon atoms
R9 represents an alkyl group or a benzylalkyl group having three to eighteen carbon atoms

n is an integer from 2 to 12

(4a) 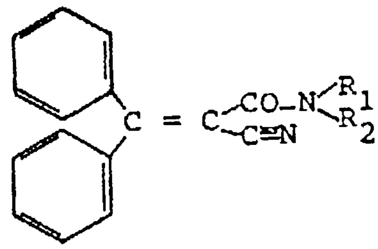

wherein R1 and R2 have the same meaning as in claim 1.

**4.** Products according to any one of claims 1 to 3 having a chromophore coupled to an amide structure of which the amine is of amino-acid or peptide origin, also having sulphur-containing groupings, these structures allowing very good fixing in the region of the skin, in particular in the region of the epidermis.

**5.** Products according to any one of claims 1 to 2, characterised in that they correspond to formulae 2a, 2b, 3a and in that they have a chromophore coupled to an amide structure of which the carbonyl is of amino-acid or peptide origin, these structures allowing very good fixing in the region of the skin, in particular in the region of the epidermis, for example with pyroglutamic acid

(2a) 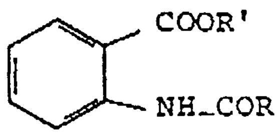

(2b) 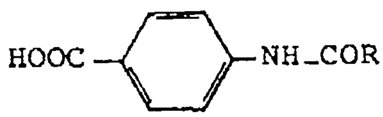

(3a) 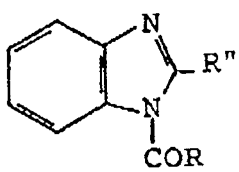

wherein R, R' and R" have the same meaning as in claim 1.

**6.** Products according to any one of claims 1 to 2 and 5 having a chromophore coupled to an amide structure of which the carbonyl is of amino-acid or peptide origin, also having sulphur-containing groupings, these structures allowing very good fixing in the region of the skin, in particular in the region of the epidermis.

**7.** Products according to any one of claims 1 to 2, characterised in that they correspond to formulae 1d and 1e and have a double amide structure of anthranilic acid or para-aminobenzoic acid at the origin of very good fixing in the region of the skin, in particular in the region of the epidermis

(1d) 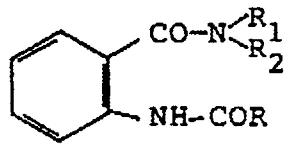

(1e) 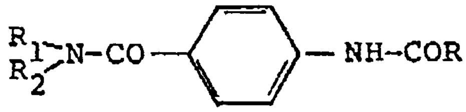

wherein R1, R2, R have the same meaning as in claim 1.

**8.** Amides according to claim 1, formed by the combination between para-methoxycinnamic acid and urocanic acid or one of its derivatives of one of the two following forms:

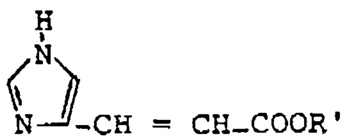

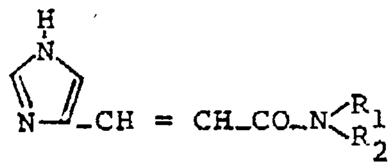

wherein R' and

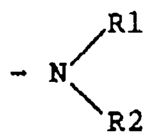

have the same meaning as in claim 1.

**9.** Products according to claim 8 having two coupled chromophore groupings and possessing water-soluble properties.

**10.** Structures according to any one of claims 1 to 9 containing salified aminoalkyl or aminoaryl groupings, for example in the form of chlorohydrates or organic or mineral salts of the free carboxylic function to add a fourth property of water-solubility, the other compounds instead being liposoluble.

**11.** Process for obtaining derivatives according to any one of claims 1 to 4, characterised in that it involves reacting anthranilic acid chloride or para-aminobenzoic acid chloride or salicylic acid chloride or 3-phenyl-2-cyano cinnamic acid chloride on an amine function of an amino-acid or of a peptide which may or may not contain sulphur.

**12.** Process for obtaining derivatives according to any one of claims 1 to 10, characterized in that it involves directly obtaining the amide bond by combination between the carboxylic function and the amine function in the presence of dicyclohexylcarbodiimide or of a similar reagent used in peptide synthesis.

**13.** Use of the compounds according to any one of claims 1 to 10 as a sun filter which selectively absorbs UVB and/or UVA radiation, fixes itself in the region of the keratins in the case of derivatives of cysteine, cystine, methionine and other sulphur-containing amino-acids and peptides and can induce melanogenesis and can therefore have a triple role, that is selectively filtering UVB and/or UVA radiation, fixing itself in the region of the epidermis of the skin and increasing to a greater or lesser extent the quantity of melanins, these substances forming the basis of the main natural system for protection against the sun.

**14.** Use of the compounds according to any one of claims 1 to 10 as tanning accelerators.

**15.** Use of the compounds according to any one of claims 1 to 10 as an active ingredient as dermo-pharmaceutical or cosmetological compositions in combination with an appropriate vehicle, for example solution, emulsion, spray, the product being applied to the surface of man's skin.

**16.** Use of the compounds according to any one of claims 1 to 10 as light screens for the perfumery and cosmetic industry or for the pharmaceutical industry for medication used for photodermatoses, in particular

- topical medication containing psoralens or derivatives thereof, salicylanilides or derivatives thereof, sulphonamides or derivatives thereof, phenothiazines, dyes or colorants such as a Bengal pink, coal tar or certain derivatives thereof and finally various products such as quinine, tretinoin, benzoyl peroxide.
- generally administered medication used for photodermatoses such as psoralens, tetracyclines, sulphonamides, sulphonyl ureas such as tolbutamide, diuretic thiazides and sulphamides, nalidixic acid, tricyclic anti-depressants such as amitriptyline and various compounds such as amiodarone, griseofulvin, quinidine, methotrexate, dicarbazine, 5 fluorouracil and vinblastine.

**Patentansprüche**

**1.** Aromatische Verbindungen, substituiert mit einer Amidfunktion, die UVB und/oder UVA absorbieren, dadurch gekennzeichnet, daß sie den Formeln 1 bis 3 entsprechen:

(1) 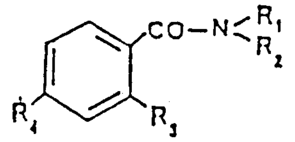

(2) 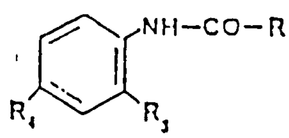

(3) 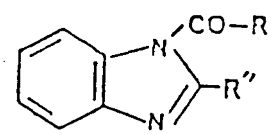

und daß man durch Einführung einer ethylenischen Bindung zwischen der aromatischen Struktur und der Amidfunktion ohne Veränderung der spektralen Charakteristika die von der Formel 4 abgeleiteten Verbindungen erhält,

(4) 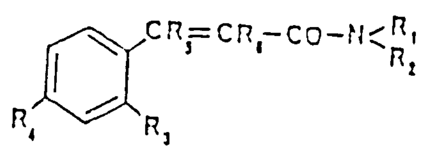

wobei diese Verbindungen wenigstens einen Chromophor aufweisen, der an eine Amidstruktur gekoppelt ist, deren Amin- oder Carbonylfunktion von einer Aminosäure oder einem Peptid stammt, derart, daß eine sehr gute Haftung auf der Hautoberfläche, insbesondere der Epidermisoberfläche, möglich wird, und sie wasserlösliche und die Melanogenese induzierende Eigenschaften besitzen können,
R1 Wasserstoff, eine Alkyl- oder eine eine Aryl-Gruppe sein kann,
R2 sein kann eine Gruppe

der Formel 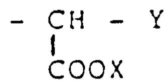

wobei X und Y Wasserstoff, eine Alkyl-, eine Aryl-, eine Aminoalkyl- oder eine Aminoaryl-Gruppe sein können,

der Formel 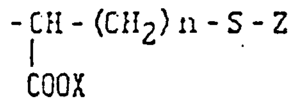

wobei Z Wasserstoff, eine Alkyl-, eine Aryl-, eine Aminoalkyl- oder eine Aminoaryl-Gruppe,
X Wasserstoff, eine Alkyl-, eine Aryl-, eine Aminoalkyl- oder eine Aminoaryl-Gruppe sein kann,
n von 1 bis 6 geht, vorzugsweise 1 oder 2 ist,

der Formel 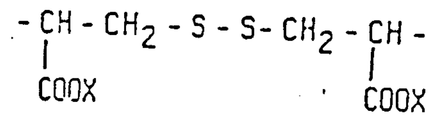

an der zwei Moleküle von durch die Formeln 1 bis 4 dargestellten filternden Chromophoren gebunden sind,
wobei X Wasserstoff, eine Alkyl-, eine Aryl-, eine Aminoalkyl- oder eine Aminoaryl-Gruppe sein oder in Form eines Mineral- oder organischen Salzes vorliegen kann,
R1 auch mit R2 identisch sein kann,

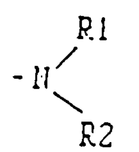

Urocansäure oder ein Derivat der Urocansäure von einem der vier folgenden Typen sein kann:

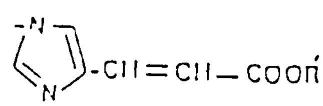

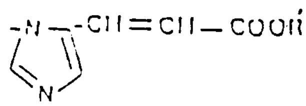

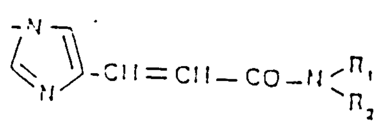

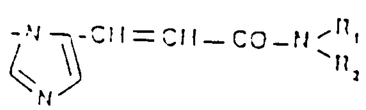

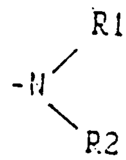

eine Peptidstruktur mit zwei oder mehreren Aminosäuren sein kann, deren terminale oder in Seitenketten befindlichen Säure- oder Aminofunktionen frei oder in Ester- oder Amidgruppen gebunden sein können,
wobei R3 und R4 Wasserstoff, eine Alkoxygruppe vom Typ $CH_3O$-, eine Hydroxyl-, eine Säure-, eine primäre Aminogruppe, eine Amidgruppe -NH-COR, eine Ester-Gruppe vom Typ -COOR' sein können,
R3 und R4 gleich oder verschieden sein können:
R entweder

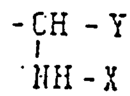

oder

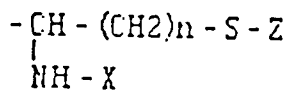

oder

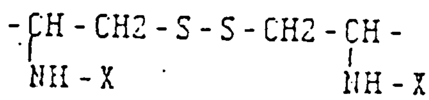

sein kann,
Z Wasserstoff, eine Alkyl-, eine Aryl-Gruppe sein kann,
n von 1 bis 6 geht, vorzugsweise 1 oder 2 ist,
X und Y Wasserstoff, eine lineare, eine verzweigte oder eventuell eine hydroxylierte Alkylgruppe, eine Aryl-, eine Aminoalkyl- oder eine Aminoaryl-Gruppe sein können,
R' Wasserstoff, eine Alkyl-, eine Aryl-, eine Aminoalkyl-, eine Aminoaryl-Gruppe oder ein organisches oder Mineralsalz sein kann,
R" eine Alkyl-, eine Aryl-, eine Aminoalkyl- oder eine Aminoaryl-Gruppe sein kann,
R5 und R6 Wasserstoff, eine Alkyl- oder Aryl-Gruppe sein können,
R5 und R6 gleich oder verschieden sein können,
wobei für die Struktur 4 die folgenden Derivate auszuschließen sind:

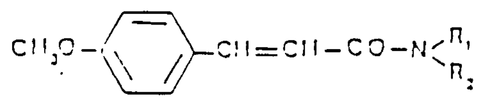

bei denen R1 und R2 dieselbe Bedeutung haben wie für die Verbindungen der Formeln 1 bis 4.

2. Amide nach Anspruch 1 infolge einer Verbindung zwischen Anthranilinsäure oder p-Aminobenzoesäure oder Salicylsäure oder 2-Cyano-3-phenyl-Zimtsäure und Aminosäuren oder Peptiden, die keinen Schwefel enthalten, wie zum Beispiel Tyrosin, Histidin, Glutamin- und Pyroglutaminsäure oder Aminosäuren und Peptiden, die Schwefel enthalten, wie Methionin, Cystein, S-Methylcystein, S-Benzylcystein, Cystin, Glutathion und oxidiertem Glutathion.

3. Amide nach einem der Ansprüche 1 oder 2 entsprechend der Formeln 1a, 1b, 1c und 4a, infolge der Verbindung zwischen Anthranilin- oder p-Aminobenzoeäure oder Salicyl- oder 2-Cyano-3-phenyl-Zimtsäure oder p-Methoxyzimtsäure und einer Aminofunktion einer Aminosäure oder eines Peptides

(1a) 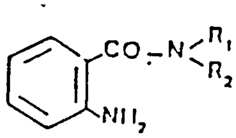

unter Ausschluß des Derivates des Methylesters des Glycin, R1 = H und R2 = $-CH_2-COOCH_3$ und unter Ausschluß des Derivates des DL-Alanin, R1 = H und

R2 = 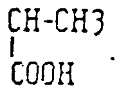

(1b) 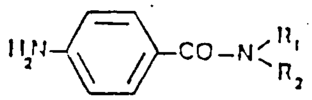

unter Ausschluß der Derivate, bei denen -NR1R2 einer Struktur $-NH-CH_2-COX'$ des Glycin oder einer Struktur

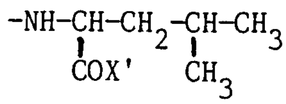

des Leucin entspricht, wobei X' einem Hydroxyl oder einer Peptidstruktur entspricht, und unter Ausschluß des Derivates des DL-Alanin, R1 = H und R2 = $-CH(COOH)-CH_3$ und der Derivate, bei denen DL-Alanin entweder durch DL-Valin, DL-Isoleucin, DL-Serin, DL-Methionin oder Asparaginsäure ersetzt ist,

(1c) 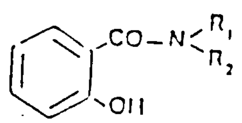

unter Ausschluß der Derivate, bei denen -NR1R2 einer Struktur $-NH-CH_2-COOX$ mit X = H (von Glycin abgeleitet) oder X = $C_2H_5$ (abgeleitet vom Ethylester des Glycin) entspricht,und unter Ausschluß der Derivate, bei denen

R1 = H und R2 = 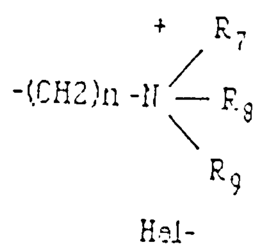

R1 = H und R2 = $-C_6H_5$,
wobei $Hal^-$ ein Halogenatom bezeichnet, $Cl^-$, $Br^-$,
wobei R7 eine Alkylgruppe mit fünf Kohlenstoffen,
R8 eine Alkylgruppe mit fünf Kohlenstoffatomen,
R9 eine Alkylgruppe oder eine Benzylalkyl-Gruppe mit 3 bis 18 Kohlenstoffatomen bezeichnet,
und n eine ganze Zahl von 2 bis 12 ist,

(4a) 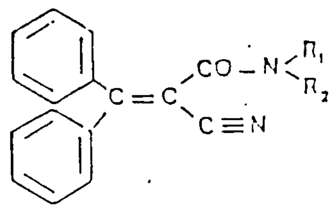

wobei R1 und R2 die gleiche Bedeutung haben wie in Anspruch 1.

**4.** Produkte nach einem der Ansprüche 1 bis 3, die einen an eine Amidstruktur, deren Amin aus einer Aminosäure oder einem Peptid stammt, das außerdem schwefelhaltige Gruppierungen aufweist, gekoppelten Chromophor aufweisen, wobei diese Strukturen eine sehr gute Haftung auf der Hautoberfläche, insbesondere auf der Epidermis-Oberfläche zuläßt.

**5.** Produkte nach einem der Ansprüche 1 oder 2, dadurch gekennzeichnet, daß sie den Formeln 2a, 2b, 3a entsprechen und einen an eine Amidstruktur, deren Carbonylgruppe aus einer Aminosäure oder einem Peptid stammt, gekoppelten Chromophor aufweisen, wobei diese Strukturen eine sehr gute Haftung auf der Hautoberfläche, insbesondere der Epidermis-Oberfläche zuläßt, zum Beispiel mit Pyroglutaminsäure

(2a) 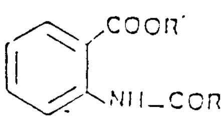

(2b) 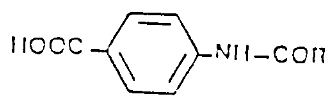

(3a) 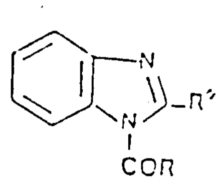

bei denen R, R' und R'' dieselbe Bedeutung haben wie in Anspruch 1.

**6.** Produkte nach einem der Ansprüche 1, 2 oder 5, die einen an eine Amidstruktur, deren Carbonylgruppe aus schwefelhaltige Gruppen aufweisenden Aminosäuren oder Peptiden stammt, gekoppelten Chromophor aufweisen, wobei diese Strukturen eine sehr gute Haftung auf der Hautoberfläche, insbesondere der Epidermis-Oberfläche zuläßt.

**7.** Produkte nach einem der Ansprüche 1 oder 2, dadurch gekennzeichnet, daß sie den Formeln 1d und 1e entsprechen und zwei Amidfunktionen der Anthranilin- oder p-Aminobenzoesäure zwecks einer sehr guten Haftung auf der Hautoberfläche, insbesondere der Epidermis-Oberfläche, aufweisen

(1d) 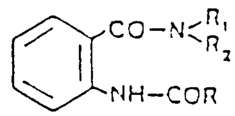

(1e) 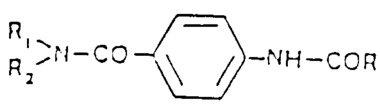

bei denen R1, R2, R die gleiche Bedeutung haben wie in Anspruch 1.

**8.** Amide nach Anspruch 1 infolge der Verbindung zwischen p-Methoxy-Zimtsäure und Urocansäure oder einem ihrer Derivate der folgenden beiden Formeln:

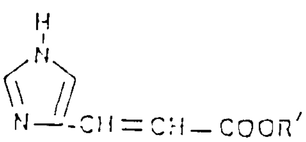

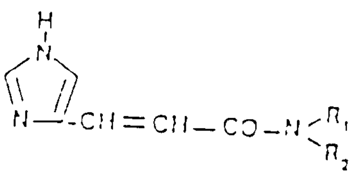

wobei R' und -NR1R2 die gleiche Bedeutung haben wie in Anspruch 1.

**9.** Produkte nach Anspruch 8, die zwei chromophore Gruppen gekoppelt aufweisen und wasserlösliche Eigenschaften besitzen.

**10.** Strukturen nach einem der Ansprüche 1 bis 9, die Aminoalkyl- oder Aminoaryl-Salzgruppen, zum Beispiel in Form von Chlorhydraten, organischen oder Mineralsalzen der freien Carboxylfunktion, enthalten, um eine vierte wasserlösliche Funktion hinzuzufügen, wobei die anderen Bestandteile eher fettlöslich sind.

**11.** Verfahren zur Herstellung der Derivate nach einem der Ansprüche 1 bis 4, dadurch gekennzeichnet, daß es daraus besteht, das Anthranilinsäurechlorid oder das p-Aminobenzoesäurechlorid oder das Salicylsäurechlorid oder das 2-Cyano-3-phenyl-Zimtsäurechlorid mit einer Aminofunktion einer schwefelhaltigen oder schwefelfreien Aminosäure oder Peptid reagieren zu lassen.

**12.** Verfahren zur Herstellung der Derivate nach einem der Ansprüche 1 bis 10, dadurch gekennzeichnet, daß es daraus besteht, die amidische Bindung durch Verbindung zwischen der Carboxylfunktion und der Aminofunktion in Gegenwart von Dicyclohexylcarbodiimid oder eines ähnlichen, bei der Peptidsynthese verwendeten Reagenzes direkt zu erhalten.

**13.** Verwendung der Verbindungen nach einem der Ansprüche 1 bis 10 als Sonnenfilter, das selektiv die UVB- und/oder UVA-Strahlung absorbiert, wobei sie auf der Ebene des Keratins die Derivate des Cystein, des Cystin, des Methionin und anderer schwefelhaltiger Aminosäuren und Peptide binden und die Melanogenese induzieren können und deshalb eine dreifache Funktion besitzen, die UVB- und/oder UVA-Strahlung selektiv filtern zu können, auf der Epidermis-Oberfläche zu binden, und die Menge der Melanine, den Grundsubstanzen des wichtigsten natürlichen Sonnenschutzsystems, mehr oder weniger stark zu erhöhen.

**14.** Verwendung der Verbindungen nach einem der Ansprüche 1 bis 10 als Bräunungsbeschleuniger.

**15.** Verwendung der Verbindungen nach einem der Ansprüche 1 bis 10 als aktive Bestandteile in dermopharmazeutischen oder kosmetischen Zusammensetzungen in Verbindung mit einem geeigneten Träger, zum Beispiel einer Lösung, Emulsion oder Spray, die alle auf der Oberfläche der menschlichen Haut anzuwenden sind.

**16.** Verwendung der Verbindungen nach einem der Ansprüche 1 bis 10 als Lichtschutz in der Parfum- und Kosmetik-Industrie oder in der pharmazeutischen Industrie als Medikamente für beginnende Photodermatosen, namentlich:

- die topischen Medikamente, die Psoralene oder ihre Derivate, Salicylanilide oder ihre Derivate, Sulfonamide oder ihre Derivate, Phenothiazine, Tinkturen oder Farbstoffe wie Bengalrosa, Steinkohlenteer oder bestimmte ihrer Derivate und schließlich verschiedene Produkte wie Chinin, Tretinoin, Benzoylperoxid... enthalten,
- die auf allgemeinem Weg bei beginnenden Photodermatosen verabreichten Medikamente wie die Psoralene, die Tetracycline, die Sulfonamide, die Sulfonylharnstoffe wie Tolbutamid, die Thiazide und diuretischen Sulfamine, Nalidixinsäure, die trizyklischen Antidepressiva wie Amitriptylin und verschiedene Verbindungen wie Amiodaron, Griseofulvin, Chinidin, Methotrexat, Dicarbazin, 5-Fluoruracil und Vinblastin.